# EUROPÄISCHE PATENTSCHRIFT

(11) **EP 1 219 260 B1**
(45) Veröffentlichungstag und Bekanntmachung des Hinweises auf die Patenterteilung: **25.06.2003**
(21) Anmeldenummer: 00127288.9
(22) Anmeldetag: 19.12.2000
(51) Int. Cl.: A61C 1/08

(54) **Verfahren und Vorrichtung zur navigationsgestüzten Zahnbehandlung**
Method and device for dental treatment assisted by a navigation system
Procédé et dispositif de traitement dentaire assisté par un système de navigation

(43) Veröffentlichungstag der Anmeldung: 03.07.2002
(73) Patentinhaber: BrainLAB AG, 85551 Kirchheim/Heimstetten (DE)
(72) Erfinder: Vilsmeier, Stefan, 6330 Kufstein (AT); Birkenbach, Rainer, 85586 Poing (DE)
(74) Vertreter: Schwabe - Sandmair - Marx

(56) Entgegenhaltungen:
- WO-A-99/16380
- DE-A- 19 902 273
- US-A- 5 846 081

## Beschreibung

Die vorliegende Erfindung betrifft ein Verfahren und eine Vorrichtung zur navigationsgestützten Zahnbehandlung gemäß dem Patentanspruch 1 sowie dem Oberbegriff des Patentanspruchs 13. Sie betrifft ferner die Verwendung einer erfindungsgemäßen Vorrichtung zur Unterstützung einer Zahnbehandlung.

Herkömmlicherweise finden Zahnbehandlungen, insbesondere invasive Zahnbehandlungen, bis heute meist noch ohne Zuhilfenahme einer Bildunterstützung bzw. Navigation statt. Meist verlässt sich ein Zahnarzt auf das, was er im Mund des Patienten sieht und auf seine Erfahrung, wenn er invasive Maßnahmen durchführt. Zur Unterstützung werden, insbesondere bei Kieferbehandlungen, oftmals noch Röntgenaufnahmen herangezogen.

Dieses Vorgehen hat einige grundsätzliche Nachteile. Unerfahrene Zahnärzte könnten beispielsweise beim Anbohren des Kiefers zum Einsetzen eines Implantatadapters in einer falschen Richtung oder zu weit bohren bzw. fräsen, was im ungünstigsten Fall Nervenschädigungen nach sich zieht. Die verwendeten Röntgenbilder sind des öfteren verzerrt und bringen damit Ungenauigkeiten ein. Die Information aus dem Röntgenbild wird nur dadurch verwertet, dass sich der Arzt die Anatomie merkt, und auch hierbei entstehen Fehlerquellen. Die Verwendung von Bohrschablonen ist teuer und verlangsamt die Behandlung.

Aus der US-A-5,562,448 ist ein Verfahren zur Vereinfachung einer zahnärztlichen Diagnose und Behandlung bekannt, bei dem die Oberfläche einer Zahnstruktur bzw. einer Kieferstruktur mit einem Instrument abgetastet wird, währen die Bewegung eines zweiten, parallel dazu angeordneten und dieselbe Bewegung durchführenden Elementes, eines sogenannten Pantograph-Armes, über Kameras verfolgt wird. Zu Diagnosezwecken wird hierbei die Oberfläche eines Zahns abgefahren und datenmäßig erfasst. Zur weiteren Unterstützung werden noch Informationen aus Röntgenbildern zusätzlich verarbeitet und integriert. Es können mehrere und unterschiedliche Pantograph-Einheiten Verwendung finden, und es ist vorgesehen, die Einrichtung gemäß der US-A-5,562,448 zur Behandlungsunterstützung und insbesondere für Übungsoperationen zu verwenden.

Der grundsätzliche Nachteil des oben genannten Systems liegt darin, dass nur Oberflächendaten zur Behandlungsunterstützung bereitstehen. Die zu behandelnden Stellen liegen nämlich oft im Zahninneren bzw. im Inneren des Kieferknochens, und mit dem hier vorgeschlagenen Verfahren ist es nicht möglich, genaue Daten über solche internen Strukturen zu erhalten. Besonders deutlich wird dies, wenn eine Kieferbehandlung dort durchgeführt werden soll, wo ein Zahn fehlt. Die Oberfläche des Kieferknochens selbst lässt sich nämlich eventuell nach invasiven Eingriffen noch bestimmen, jedoch ist seine innere Struktur und Lage durch dieses System nicht erfassbar. Außerdem ist die Bereitstellung eines Behandlungsoder Abtastgerätes mit einem daran über eine Befestigung im Abstand angebrachten parallelen Pantograph-Arm aufwändig und es entsteht ein sperriges Instrument, dass noch an einem Gelenkarm befestigt werden muss, um überhaupt handhabbar und funktionsfähig zu sein.

Dokument DE 199 02 273 offenbart ein Verfahren zur Unterstützung einer Zahnbehandlung dem Oberbegriff des Anspruchs 1 entsprechend sowie eine Vorrichtung gemäß dem Oberbegriff des Anspruch 14.

Es ist die Aufgabe der vorliegenden Erfindung, ein Verfahren und eine Vorrichtung zur navigationsgestützten Zahnbehandlung bereitzustellen, welche die oben angeführten Probleme des Standes der Technik überwinden. Insbesondere soll ein ausreichend handhabungsfähiges, navigationsgestütztes Zahnbehandlungssystem vorgeschlagen werden, welches optimal genaue Behandlungen bzw. Eingriffe gestattet. Diese Aufgabe wird gemäß der vorliegenden Erfindung gelöst durch ein Verfahren zur Unterstützung einer Zahnbehandlung, bei dem
- mittels eines bildgebenden Verfahrens, beispielsweise einer Tomographieerfassung, ein dreidimensionaler Volumendatensatz für den Zahn- bzw. Kieferbereich eines Patienten erstellt wird,
- der Zahn- bzw. Kieferbereich mittels einer computergestützten Navigations- bzw. Trackingsystems, welches den Volumendatensatz verwendet, registriert bzw. referenziert wird,
- Behandlungsinstrumente bzw. -apparaturen gegenüber dem computergesteuerten Navigations- bzw. Trackingsystem registriert bzw. referenziert und positionell verfolgt werden, und bei dem
- das Navigations- bzw. Trackingsystem mittels einer Ausgabeeinheit vor, während oder nach der Behandlung behandlungsunterstützende bzw. behandlungsleitende Informationen liefert.

Der Vorteil der vorliegenden Erfindung beruht insbesondere auf der Bereitstellung eines dreidimensionalen Volumendatensatzes für den Zahn- bzw. Kieferbereich eines Patienten. Mit einer solchen Information, die beispielsweise durch ein Tomographieverfahren erhalten werden kann, stehen dem behandelnden Arzt nunmehr nicht mehr lediglich nur Oberflächeninformationen sondern auch Informationen über die innere Struktur von Zahn bzw. Kiefer zur Verfügung, und er kann seine Operationstätigkeit auf der Basis dieser Informationen durchführen. Hierdurch wird sichergestellt, dass gesunde Gewebe- bzw. Knochenstrukturen weitgehend geschont werden können, während kranke oder veränderte Strukturen vollständig abgetragen werden können. Insbesondere im Rahmen der zahnärztlichen Implantologie ermöglicht es die vorliegende Erfindung, Bohrungen oder Einfräsungen in falschen Richtungen auszuschließen und, weil durch die Navigation bzw. das Tracking immer Informationen über die Lage der Bohrer- oder Fräserspitze vorliegen, ist es auch möglich, positiv zu verhindern, dass zu weit oder zu wenig weit eingebohrt bzw. eingefräst wird, so dass Nervenschädigungen bzw. ein schlechter Halt eines Zahnimplantat-Adapters vermieden werden können. Dem Navigations- bzw. Trackingsystem ist gemäß der vorliegenden Erfindung zu jedem Zeitpunkt die Lage der relevanten Patiententeile sowie die Lage der Instrumente bekannt und all diese Daten können auf einem Bildschirm ausgegeben werden und dem Arzt bei der Behandlung behilflich sein. Insgesamt wird die Behandlung dadurch genauer; bei der Anbringung von Zahnimplantat-Adaptern kann dies soweit gehen, dass der Zahnersatz selbst schon in einer vorbestimmten Lage auf den Adapter aufgesetzt wird, ohne dass die bisher verwendeten Kugelgelenke zur Nachjustierung notwendig sind.

Die Vorrichtung gemäß der vorliegenden Erfindung weist eine Einrichtung auf, die mittels eines bildgebenden Verfahrens, beispielsweise einer Tomographieerfassung, einen dreidimensionalen Volumensatz für den Zahn- bzw. Kieferbereich eines Patienten erstellt, und die den Zahn- bzw. Kieferbereich mittels des Navigations- bzw. Trackingsystems, welches den Volumendatensatz verwendet, registriert bzw. referenziert.

Im Rahmen der vorliegenden Erfindung wird grundsätzlich die Position der Behandlungsinstrumente bzw. -apparaturen durch das Navigations- bzw. Trackingsystem verfolgt. Dies kann genügen, wenn der Kopf des Patienten ausreichend fixiert ist und die Lage der Fixierungseinrichtung gegenüber dem Navigations- bzw. Trackingsystem feststeht. Dies gilt vor allem für Behandlungen im Oberkieferbereich. Es besteht aber grundsätzlich die Möglichkeit, auch den Zahn- bzw. Kieferbereich durch das Navigations- bzw. Trackingsystem positionell zu verfolgen, um immer aktuelle Daten über die Lage des zu behandelnden Gebietes zur Verfügung zu haben. Empfehlenswert ist eine solche positionelle Verfolgung des Zahn- bzw. Kieferbereiches insbesondere bei nicht fixierten Patienten oder bei Behandlungen am Unterkiefer.

Bei einer bevorzugten Ausführungsform der Erfindung wird bei der Behandlung auf einer Bildschirmausgabe der Zahn- bzw. Kieferbereich sowie der vorab oder interoperativ geplante Sollpositionsverlauf eines Instruments aufgezeigt und der Istpositionsverlauf des Instruments positionell verfolgt. Bei Abweichungen zwischen dem Sollpositionsverlauf und dem Istpositionsverlauf kann eine visuelle und/oder akustische Warnung ausgegeben werden, so dass der Arzt entsprechende Korrekturen vornehmen kann. Um auch solche Korrekturen unterstützen zu können, kann bei festgestellten Abweichungen zwischen dem Sollpositionsverlauf und dem Istpositionsverlauf durch das computergesteuerte Navigations- und Trackingsystem ein neuer Sollpositionsverlauf errechnet und ausgegeben werden, dem der Arzt danach folgen kann.

Im Rahmen der vorliegenden Erfindung kann ein Navigations- und Trackingsystem verwendet werden, bei welchem die Registrierung bzw. Referenzierung eines dreidimensonale Daten umfassenden Datensatzes für den Zahn- und Kieferbereich mittels einer der folgenden Methoden durchgeführt wird:
- Referenzierung bzw. Registrierung des Datensatzes mittels anatomischer Landmarken,
- Referenzierung bzw. Registrierung des Datensatzes mittels künstlicher Landmarken, insbesondere aktiv abstrahlender oder reflektierender Marker,
- Referenzierung bzw. Registrierung des Datensatzes mittels einer Surface-Matching-Technologie. die auf anatomischer Abtastung beruht,
- Referenzierung bzw. Registrierung des Datensatzes mittels einer Surface-Matching-Technologie, die auf berührungsfreier Abtastung beruht, insbesondere auf der Erzeugung von erfassbaren Laserstrahlmarkierungen auf der Patientenoberfläche.

Auch Kombinationen sind möglich.

Die positionelle Verfolgung des Zahn- bzw. Kieferbereiches kann vorteilhafterweise über eine dynamische Trackingmethode erfolgen, insbesondere durch ein gleichzeitiges dynamisches Tracking des Oberkiefers und/oder des Unterkiefers bzw. des Oberkiefers und des Unterkiefers jeweils einzeln, bevorzugt über dort befestigte Referenzierungsadapter mit Markeranordnungen.

Als Navigations- bzw. Trackingsystem können bekannte Systeme verwendet werden, die beispielsweise in der Stereotoxie heute schon eingesetzt werden. So steht beispielsweise ein optisches Navigations- und Trackingsystem zur Verfügung, welches die Registrierung bzw. Referenzierung und die positionelle Verfolgung des Zahn- bzw. Kieferbereiches und/oder der Behandlungsinstrumente bzw. -apparaturen unter Verwendung von Markern bzw. Markeranordnungen und eines Kamerasystems durchführt. Insbesondere eignet sich auch ein Infrarot-Navigations- bzw. Trackingsystem als optisches System, bei dem die Marker als Reflektoren ausgebildet sind. Ebenfalls besteht die Option, im Rahmen der Erfindung ein magnetfeldbasiertes Navigations- und Trackingsystem zu verwenden, welches die Registrierung bzw. Referenzierung und die positionelle Verfolgung des Zahn- bzw. Kieferbereiches und/oder der Behandlungsinstrumente bzw. -apparaturen unter Verwendung eines äußeren Magnetfeldes und mit induktionssensitiven Elementen durchführt.

Wie oben schon angesprochen können Kombinationen dieser Navigations- und Trackingsysteme verwendet werden, die untereinander abgestimmt sind. So wird gemäß einer vorteilhaften Ausführungsform der Erfindung ein optisches Navigations- und Trackingsystem verwendet, welches die Registrierung bzw. Referenzierung und die positionelle Verfolgung des Zahn- bzw. Kieferbereiches durchführt. Zusätzlich wird bei dieser Ausführungsform ein bezüglich der Navigation bzw. des Trackings hieran gekoppeltes magnetfeldbasiertes Navigations- und Trackingsystem verwendet, welches die Registrierung bzw. die Referenzierung und die positionelle Verfolgung der Behandlungsinstrumente bzw. -apparaturen unter Verwendung eines äußeren Magnetfeldes und induktionssensitiven Elementen durchführt.

Der dreidimensionale Volumendatensatz, auf dessen Basis die Behandlung stattfindet, kann beispielsweise durch Computertomographie, Kernspintomographie, PET, SPECT, Ultraschallerfassung und ähnliche Verfahren und durch eine Kombination solcher Verfahren erhalten werden. Wenn die Behandlung zusätzlich durch intraoperative bildgebende Verfahren unterstützt wird, eröffnet sich die Möglichkeit, durch den Eingriff veränderte Strukturen schon während der Behandlung in der Navigation zu berücksichtigen. Dies ist dann von besonderem Vorteil, wenn der Mund des Patienten offen fixiert ist und momentan keine Möglichkeit besteht, den Bisszustand durch ein tatsächliches Aufeinanderbeißen zu überprüfen. Das System, welches durch die intraoperativen bildgebenden Verfahren, den momentanen Ist-Zustand des Patienten kennt, kann zum Beispiel ein Schließen des Kiefers virtuell simulieren und dabei notwendige Nachbehandlungen aufzeigen, ohne dass der Patient selbst aus der Fixierung gelöst werden muss.

Die Bildschirmausgabe kann erfindungsgemäß im Rahmen der folgenden Darstellungen erfolgen:
- zwei- und dreidimensionale Darstellung des registrierten bzw. referenzierten und positionsbestimmten Zahn- bzw. Kieferbereiches sowie der Instrumente mit Soll- und Istpositionsverlauf (es können lediglich zweidimensionale, lediglich dreidimensionale oder auch kombinierte Darstellungsweisen gewählt werden),
- OPG-Ansichten,
- digitale rekonstruierte Röntgenbilder (DRR = Digitally Reconstructed Radiographs), insbesondere in Form von OPG-Ansichten.

Die vorliegende Erfindung zieht auch die Verwendung von Robotertechnologie in Betracht. So kann die Behandlung durch Instrumente erfolgen, die vollständig oder teilweise durch einen Roboter geführt werden, der im Navigations- bzw. Trackingsystem registriert bzw. referenziert und positionell verfolgt wird und der durch das System angesteuert wird. Es ist in diesem Rahmen möglich, beispielsweise einen Bohrer oder einen Fräser von einem Roboterarm über einer bestimmten Stelle im Kieferknochen zu positionieren. Das Bohren bzw. Fräsen kann dann ebenfalls durch den Roboter erledigt werden oder aber auch durch die Führung des Instrumentes am Roboterarm durch den Arzt, wobei der Roboter richtige Bewegungen gestattet und falsche Bewegungen verhindert.

Die erfindungsgemäße Vorrichtung und ihre Ausführungsformen sind in den Ansprüchen dargelegt. Mit dieser Vorrichtung lassen sich ebenfalls die schon oben erwähnten Vorteile erzielen.

Ferner betrifft die Erfindung noch die Verwendung einer solchen Vorrichtung zur Unterstützung einer Zahnbehandlung.

Die Erfindung wird im Weiteren anhand der beiliegenden Zeichnungen sowie anhand eines Ausführungsbeispieles näher erläutert. Es zeigen:
- Figur 1: ein Navigations- und Trackingsystem, das im Rahmen der vorliegenden Erfindung eingesetzt werden kann;
- Figur 2: einen navigierten bzw. getrackten Fräser beim Anbohren eines Kieferknochens; und
- Figur 3: ein eingesetztes Zahnimplantat.

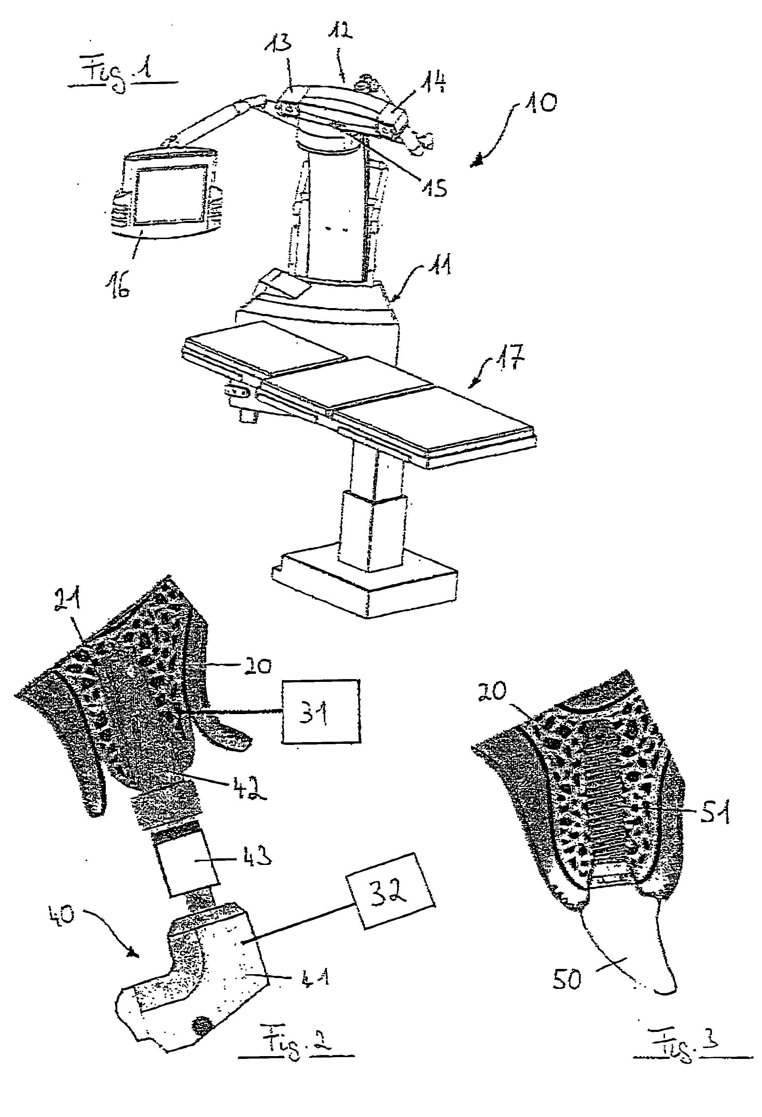

Die Figur 1 zeigt ein Navigations- bzw. Trackingsystem, wie es im Rahmen der vorliegenden Erfindung zur Unterstützung einer Zahnbehandlung verwendet werden kann. Das System ist insgesamt mit dem Bezugszeichen 10 bezeichnet, und es weist eine Patientenliege 17 auf, auf der ein Patient zur Behandlung positioniert werden kann. Anstelle der Patientenliege 17 kann beispielsweise auch ein Zahnarztstuhl verwendet werden.

Das System umfasst weiter ein verfahrbares Gerät, an dessen Basis sich ein Computer 11 befindet, der alle Steuerungen und Regelungen übernimmt sowie die Verarbeitung der empfangenen und der auszugebenden Signale. Ein Kameraträger 12, der an einem Arm am System befestigt ist, weist zwei Infrarotkameras 13 und 14 auf, sowie einen Infrarotsender 15. Mit diesen Kameras lassen sich Marker oder Markeranordnungen verfolgen und positionell referenzieren bzw. registrieren, welche an dem Patienten und/oder an verschiedenen Behandlungsinstrumenten bzw. -apparaturen angebracht sind. Vorzugsweise handelt es sich dabei um Anordnungen von reflektierenden Markern, über deren Positionsbestimmung auch beispielsweise die Position der Spitze eines Instrumentes erfasst werden kann.

Als Ausgabeeinrichtung wird ein Bildschirm 16 zur Verfügung gestellt, der dem Arzt eine Reihe von zwei- bzw. dreidimensionalen Ansichten über aktuelle Instrumentenpositionen und Anatomiedaten zur Verfügung stellt. Gleichzeitig können Soll- und Istpositionsverläufe für die Instrumente eingeblendet werden. Der Bildschirm kann auch als Eingabemedium, beispielsweise zur Einstellung der gewünschten Ansichten dienen. Außerdem kann das System akustische Meldungen abgeben, beispielsweise bei Abweichungen zwischen dem errechneten optimalen und dem tatsächlichen Einbohrkanal eines Kieferbohrers.

Die vorliegende Erfindung eignet sich insbesondere für Maßnahmen im Rahmen der Kieferbehandlung und hier speziell im Rahmen der zahnärztlichen Implantologie.

In Figur 2 ist dargestellt, wie mit einem Fräser 42, der über einem Adapter 43 an seiner Halterung 41 befestigt ist, ein Verankerungsloch 21 in einem Kieferknochen 20 ausgefräst wird. Die Schleimhaut über dem Kieferknochen ist dazu entfernt worden.

Schon in der Darstellung der Figur 2 wird deutlich, dass das Loch 21 im Kieferknochen 20 einen genau bestimmten Verlauf haben muss, und zwar schon deshalb, damit auf jeder Seite um das Loch 21 herum genügend Kieferknochen übrig bleibt. Es wird nämlich, wie in Figur 3 zu sehen ist, in das Loch später ein Zahnimplantat-Adapter 51 eingesetzt, auf den das Zahnimplantat 50 aufzusetzen ist. Dieses Zahnimplantat 50 muss aber mit ausreichender Festigkeit positioniert und insbesondere soll es sich in die Ausrichtung der nebenstehenden Zähne einfügen. Deshalb ist auch die Ausrichtung des Loches 21 (Figur 2) mit äußerster Genauigkeit durchzuführen.

Diese Genauigkeit lässt sich nunmehr mit Hilfe der vorliegenden Erfindung erzielen. Zu diesem Zweck werden sowohl der Kieferknochen 20 als auch die mit den Bezugszeichen 40 insgesamt bezeichnete Fräseinrichtung in einem Navigations- bzw. Trackingsystem 10 referenziert bzw. registriert und positionell verfolgt. Dies geschieht für den Kiefer über eine fest am Kiefer angebrachte Markierung 31 und für die Fräseinrichtung über eine fest dort angebundene Markierung 32. Über diese Markierungen, deren Position im Navigationssystem 10 ständig erfassbar ist, sind auch die aktuellen Positionen der Kieferanatomie sowie des Instrumentes 40 und dessen Fräserspitze 42 zu jedem Zeitpunkt bekannt. Erfindungsgemäß sind durch die dreidimensionale Volumendatenaquisition vom Kieferbereich sowohl alle Oberflächendaten als auch alle inneren Daten bekannt und es lassen sich damit Operationen planen; überwachen und leiten, die alle äußeren und inneren Gegebenheiten der Körperstruktur des Patienten berücksichtigen.

Die Markierungen 31 und 32 können von unterschiedlicher Art sein. Für die Markierung 31 eignet sich beispielsweise eine am Kiefer befestigte Referenzierungs-Markeranordnung, die angeklammert werden kann. Ein solcher Referenzadapter weist beispielsweise drei Marker auf und kann dem Navigationssystem 10 jederzeit die Position des Körperteils mitteilen, an dem er befestigt ist.

Die Markierung 32 kann ebenfalls eine solche am Instrument befestigte Markeranordnung umfassen, wobei es ohne weiteres möglich ist, die Markeranordnung über einen Adapter beispielsweise am hinteren Ende der Fräsvorrichtung 40 anzubringen, damit sie bei der Behandlung nicht stört. Auch hier gilt dann, dass die Position der Fräseinrichtung 40 und insbesondere auch der Spitze des Fräsers zu jedem Zeitpunkt bekannt ist und im Rahmen der Behandlungsüberwachung bzw. Behandlungsleitung verwendet werden kann.

Anstelle von Markeranordnungen lassen sich auch andere bekannte Trackingsysteme einsetzen, beispielsweise magnetbasierte Systeme, bei denen im Behandlungsgebiet ein Magnetfeld erzeugt und in oder an den Instrumenten oder an dem Patienten induktionssensitive Elemente, beispielsweise Spulen positioniert werden. Demgemäß können die Markierungen 31 und 32 auch für solche Magnettracking-Elemente stehen, die geeigneterweise auch in den Gehäusen von Instrumenten anzuordnen sind. Hierbei können verschiedene Systeme zum Einsatz kommen, nämlich solche die gepulste, gleichstrominduzierte Magnetfelder verwenden, solche die Wechselstromfelder verwenden, solche die vollständig passive Empfänger verwenden und solche die aktive Empfänger verwenden. Ein großer Vorteil dieser magnetbasierten Navigations- und Trackingssysteme besteht darin, dass keine Sichtlinie zwischen den Markern 31 und 32 und einem Kamerasystem bestehen muss, was dem Arzt eine weitgehende Bewegungsfreiheit einräumt.

Eine Behandlung, beispielsweise das Einsetzen eines zahnärztlichen Implantates wird beispielsweise wie folgt ablaufen. Zunächst wird eine Tomographieerfassung für den Zahn- bzw. Kieferbereich eines Patienten erstellt, also ein dreidimensionaler Volumendatensatz, in dem alle Informationen über das Behandlungsgebiet gespeichert sind. Diese Daten werden dann dem Navigations- bzw. Trackingssystem 10 mitgeteilt, d. h. im Computer 11 gespeichert und über eine Markierung 31 erfasst das System 10 während und bei der Behandlung die aktuelle Position aller, also auch interner Strukturdaten des Zahn- bzw. Kieferbereiches des Patienten. Der Arzt kann nunmehr eingehen, beispielsweise an einem berührungsempfindlichen Bildschirm 16, wo er in etwa die Bohrung für den Implantat-Adapter 51 einbringen möchte, wobei dieser Schritt auch schon vor der Behandlung durchgeführt werden kann. Das System errechnet anhand aller zur Verfügung stehenden Daten, auch Daten über verschiedene Standard-Implantate (insbesondere auch das zu verwendende Implantat) wie genau das zu bohrende Loch zu verlaufen hat und wo es enden soll. Diese Bohrlinie kann dann auf dem Bildschirm 16 in mehreren Ansichten (zwei- oder dreidimensional) virtuell dargestellt werden. Der Arzt kann nun einen Bohrer oder Fräser in die Hand nehmen, dessen Position ebenfalls im Navigationssystem bekannt ist. Insbesondere ist beispielsweise eine Standard-Fräseinrichtung 40 zu verwenden, deren Spitzenposition (und gesamte äußere Gestalt) bekannt und über die Markierung 32 im Navigations- bzw. Trackingsystem verfolgbar ist. Unterstützt durch die Bildausgabe kann der Arzt nunmehr auf der vorgeplanten Linie bohren oder fräsen und wird bei Richtungsabweichungen oder bei zu tiefem Einbohren bzw. Einfräsen gewarnt. Es entsteht ein sauberes und genau ausgerichtetes Loch, in welches der Implantat-Adapter 51 genau eingesetzt werden kann, um dem später aufzusetzenden Zahnimplantat 50 eine vollständig richtige Positionierung zu geben.

## Patentansprüche

1. Verfahren zur Unterstützung einer Zahnbehandlung, bei dem
- mittels eines bildgebenden Verfahrens, beispielsweise einer Tomographieerfassung, ein dreidimensionaler Volumendatensatz für den Zahn- bzw. Kieferbereich eines Patienten erstellt wird,
- der Zahn- bzw. Kieferbereich mittels eines computergestützten Navigations- bzw. Trackingsystems (10), welches den Volumendatensatz verwendet, registriert bzw. referenziert wird,
- Behandlungsinstrumente bzw. -apparaturen (40) gegenüber dem computergestützten Navigations- bzw. Trackingsystem (10) registriert bzw. referenziert und positionell verfolgt werden, und bei dem
- das Navigations- bzw. Trackingsystem (10) mittels einer Ausgabeeinheit (16) vor, während oder nach der Behandlung behandlungsunterstützende bzw. behandlungsleitende Informationen liefert, **dadurch gekennzeichnet, dass**
- bei Abweichungen zwischen dem Sollpositionsverlauf und dem Istpositionsverlauf ein neuer Sollpositionsverlauf errechnet und ausgegeben wird.

2. Verfahren nach Anspruch 1, bei dem auch der Zahn- bzw. Kieferbereich durch das Navigations- bzw. Trackingsystem positionell verfolgt wird.

3. Verfahren nach Anspruch 1 oder 2, bei dem bei der Behandlung auf einer Bildschirmausgabe (16) der Zahn bzw. Kieferbereich sowie der vorab oder intraoperativ geplante Sollpositionsverlauf eines Instruments (40) aufgezeigt und der Istpositionsverlauf des Instruments (40) positionell verfolgt wird.

4. Verfahren nach Anspruch 3, bei dem bei Abweichungen zwischen dem Sollpositionsverlauf und dem Istpositionsverlauf eine visuelle und/oder akustische Warnung ausgegeben wird.

5. Verfahren nach einem der Ansprüche 1 bis 4, bei dem ein Navigations- und Trackingsystem verwendet wird, welches die Registrierung bzw. Referenzierung eines dreidimensonale Daten umfassenden Datensatzes für den Zahn- und Kieferbereich mittels einer der folgenden Methoden durchführt:
- Referenzierung bzw. Registrierung des Datensatzes mittels anatomischer Landmarken,
- Referenzierung bzw. Registrierung des Datensatzes mittels künstlicher Landmarken, insbesondere aktiv abstrahlender oder reflektierender Marker,
- Referenzierung bzw. Registrierung des Datensatzes mittels einer Surface-Matching-Technologie, die auf anatomischer Abtastung beruht,
- Referenzierung bzw. Registrierung des Datensatzes mittels einer Surface-Matching-Technologie, die auf berührungsfreier Abtastung beruht, insbesondere auf der Erzeugung von erfassbaren Laserstrahlmarkierungen auf der Patientenoberfläche.

6. Verfahren nach einem der Ansprüche 1 bis 5, bei dem die positionelle Verfolgung des Zahn- bzw. Kieferbereiches über eine dynamische Trackingmethode erfolgt, insbesondere durch ein gleichzeitiges dynamisches Tracking des Oberkiefers und/oder des Unterkiefers bzw. des Oberkiefers und des Unterkiefers jeweils einzeln, bevorzugt über dort befestigte Referenzierungsadapter mit Markeranordnungen.

7. Verfahren nach einem der Ansprüche 1 bis 6, bei dem ein optisches Navigations- und Trackingsystem verwendet wird, welches die Registrierung bzw. Referenzierung und die positionelle Verfolgung des Zahn- bzw. Kieferbereiches und/oder der Behandlungsinstrumente bzw. Apparaturen unter Verwendung von Markern bzw. Markeranordnungen (31, 32) und eines Kamerasystems durchführt, wobei insbesondere ein Infrarot-Navigations- bzw. Trackingsystem verwendet wird.

8. Verfahren nach einem der Ansprüche 1 bis 7, bei dem ein magnetfeldbasiertes Navigations- und Trackingsystem verwendet wird, welches die Registrierung bzw. Referenzierung und die positionelle Verfolgung des Zahn- bzw. Kieferbereiches und/oder der Behandlungsinstrumente bzw. Apparaturen unter Verwendung eines äußeren Magnetfeldes und induktionssensitiven Elementen (31, 32) durchführt.

9. Verfahren nach einem der Ansprüche 1 bis 8, bei dem ein optisches Navigations- und Trackingsystem verwendet wird, welches die Registrierung bzw. Referenzierung und die positionelle Verfolgung des Zahn- bzw. Kieferbereiches durchführt, und bei dem ein bezüglich der Navigation bzw. des Trackings hieran gekoppeltes magnetfeldbasiertes Navigations- und Trackingsystem verwendet wird, welches die Registrierung bzw. Referenzierung und die positionelle Verfolgung der Behandlungsinstrumente bzw. Apparaturen unter Verwendung eines äußeren Magnetfeldes und induktionssensitiven Elementen (31, 32) durchführt.

10. Verfahren nach einem der Ansprüche 1 bis 9, bei dem der dreidimensionale Volumendatensatz erhalten wird durch eines oder mehrere der folgenden Verfahren: Computertomographie, Kernspintomographie, PET, SPECT, Ultraschallerfassung.

11. Verfahren nach einem der Ansprüche 1 bis 10, bei dem die Behandlung ferner durch intraoperative bildgebende Verfahren unterstützt wird.

12. Verfahren nach einem der Ansprüche 1 bis 11, bei dem eine Bildschirmausgabe auf der Basis einer oder mehrere der folgenden Darstellungen erfolgt:
- zwei- und dreidimensionale Darstellung des registrierten bzw. referenzierten und positionsbestimmten Zahn- bzw. Kieferbereiches sowie der Instrumente mit Sollund Istpositionsverlauf,
- OPG-Ansichten,
- digital rekonstruierte Röntgenbilder (DRR = Digitally Reconstructed Radiographs), insbesondere in Form von OPG-Ansichten.

13. Verfahren nach einem der Ansprüche 1 bis 12, bei dem die Behandlung durch Instrumente erfolgt, die vollständig oder teilweise durch einen Roboter geführt werden, der im Navigations- bzw. Trackingsystem (10) registriert bzw. referenziert und positionell verfolgt wird und der durch das System angesteuert wird.

14. Vorrichtung zur Unterstützung einer Zahnbehandlung mit einem Navigations- bzw. Trackingsystem (10), das mittels einer Ausgabeeinheit (16) bei der Behandlung behandlungsunterstützende bzw. behandlungsleitende Informationen liefert und mit Behandlungsinstrumenten bzw. -apparaturen (40), die gegenüber dem computergestützten Navigations- bzw. Trackingsystem (10) registriert bzw. referenziert und positionell verfolgt werden, mit einer Einrichtung, die mittels eines bildgebenden Verfahrens, beispielsweise einer Tomographieerfassung, einen dreidimensionalen Volumendatensatz für den Zahn- bzw. Kieferbereich eines Patienten erstellt, und die den Zahn- bzw. Kieferbereich mittels des Navigations- bzw. Trackingsystems (10), welches den Volumendatensatz verwendet, registriert bzw. referenziert, **dadurch gekennzeichnet, dass** sie eine Ausgabeeinrichtung aufweist, die bei Abweichungen zwischen dem Sollpositionsverlauf und dem Istpositionsverlauf eine visuelle und/oder akustische Warnung ausgibt, worauf insbesondere bei Abweichungen zwischen dem Sollpositionsverlauf und dem Istpositionsverlauf ein neuer Sollpositionsverlauf errechnet und ausgegeben wird

15. Vorrichtung nach Anspruch 14, **dadurch gekennzeichnet, dass** sie eine Einrichtung aufweist, mit der auch der Zahn- bzw. Kieferbereich durch das Navigations- bzw. Trackingsystem positionell verfolgt wird.

16. Vorrichtung nach Anspruch 14 oder 15, **dadurch gekennzeichnet, dass** die Ausgabeeinheit eine Bildschirmausgabe (16) ist, auf der bei der Behandlung der Zahn bzw. Kieferbereich sowie der vorab oder intraoperativ geplante Sollpositionsverlauf eines Instruments (40) aufgezeigt und der Istpositionsverlauf des Instruments (40) positionell verfolgt wird.

17. Vorrichtung nach Anspruch 16, **dadurch gekennzeichnet, dass** sie ein optisches Navigations- und Trackingsystem aufweist, welches die Registrierung bzw. Referenzierung und die positionelle Verfolgung des Zahn- bzw. Kieferbereiches und/oder der Behandlungsinstrumente bzw. Apparaturen unter Verwendung von Markern bzw. Markeranordnungen (31, 32) und eines Kamerasystems durchführt, insbesondere ein Infrarot-Navigations- bzw. Trackingsystem.

18. Vorrichtung nach einem der Ansprüche 14 bis 17, **dadurch gekennzeichnet, dass** sie ein magnetfeldbasiertes Navigations- und Trackingsystem aufweist, welches die Registrierung bzw. Referenzierung und die positionelle Verfolgung des Zahn- bzw. Kieferbereiches und/oder der Behandlungsinstrumente bzw. Apparaturen unter Verwendung eines äußeren Magnetfeldes und induktionssensitiven Elementen (31, 32) durchführt.

19. Vorrichtung nach einem der Ansprüche 14 bis 18, **dadurch gekennzeichnet, dass** sie ein optisches Navigations- und Trackingsystem aufweist, welches die Registrierung bzw. Referenzierung und die positionelle Verfolgung des Zahn- bzw. Kieferbereiches durchführt, und dadurch, dass sie ein bezüglich der Navigation bzw. des Trackings hieran gekoppeltes magnetfeldbasiertes Navigations- und Trackingsystem aufweist, welches die Registrierung bzw. Referenzierung und die positionelle Verfolgung der Behandlungsinstrumente bzw. Apparaturen unter Verwendung eines äußeren Magnetfeldes und induktionssensitiven Elementen (31, 32) durchführt.

20. Vorrichtung nach einem der Ansprüche 14 bis 19, **dadurch gekennzeichnet, dass** sie ferner Einrichtungen aufweist, mit denen die Behandlung durch intraoperative bildgebende Verfahren unterstützt wird.

21. Vorrichtung nach einem der Ansprüche 14 bis 20, **dadurch gekennzeichnet, dass** sie einen Roboter aufweist, der bei der Behandlung die Instrumente die vollständig oder teilweise führt, der im Navigations- bzw. Trackingsystem (10) registriert bzw. referenziert und positionell verfolgt wird und der durch das System angesteuert wird.

## Claims

1. A method for assisting in a dental treatment, wherein:
- a three-dimensional volume data set for the tooth and/or jaw area of a patient is produced by means of an imaging method, for example tomographic imaging;
- the tooth and/or jaw area is registered and/or referenced by means of a computer-assisted navigation and/or tracking system (10) using the volume data set;
- instruments and apparatus for treatment (40) are registered and/or referenced relative to the computer-assisted navigation and/or tracking system (10), and positionally tracked; and wherein:
- the navigation and/or tracking system (10) supplies information for assisting and guiding the treatment by means of an output unit (16), before, during or after the treatment
**characterised in that**
- a new desired positional course is calculated and outputted when the desired positional course and actual positional course deviate.

2. The method as set forth in claim 1, wherein the tooth and/or jaw area is also positionally tracked by the navigation and/or tracking system.

3. The method as set forth in claim 1 or 2, wherein the tooth and/or jaw area, as well as the desired positional course of an instrument (40), planned to begin with or inter-operatively, are displayed during the treatment on a screen (16), and the actual positional course of the instrument (40) is positionally tracked.

4. The method as set forth in claim 3, wherein a visual and/or acoustic warning signal is outputted when the desired positional course and actual positional course deviate.

5. The method as set forth in any one of claims 1 to 4, wherein a navigation and/or tracking system is used which registers and/or references a data set comprising three-dimensional data for the tooth and jaw area, by means of one of the following methods:
- referencing and/or registering the data set by means of anatomical landmarks;
- referencing and/or registering the data set by means of artificial landmarks, in particular actively emitting or reflecting markers;
- referencing and/or registering the data set by means of a surface-matching technology based on anatomical scanning;
- referencing and/or registering the data set by means of a surface-matching technique based on non-contact scanning, in particular on generating detectable laser beam markings on the surface of the patient.

6. The method as set forth in any one of claims 1 to 5, wherein the tooth and/or jaw area is positionally tracked using a dynamic tracking method, in particular a simultaneous dynamic tracking of the upper jaw and/or lower jaw or of the upper jaw and lower jaw individually, preferably via referencing adaptors secured there, comprising arrangements of markers.

7. The method as set forth in any one of claims 1 to 6, wherein an optical navigation and/or tracking system is used which registers and/or references and positionally tracks the tooth and/or jaw area and/or the instruments or apparatus for treatment, using markers or arrangements of markers (31, 32) and a camera system, wherein in particular an infrared navigation and/or tracking system is used.

8. The method as set forth in any one of claims 1 to 7, wherein a magnetic field based navigation and/or tracking system is used which registers and/or references and positionally tracks the tooth and/or jaw area and/or the instruments or apparatus for treatment, using an external magnetic field and induction-sensitive elements (31, 32).

9. The method as set forth in any one of claims 1 to 8, wherein an optical navigation and/or tracking system is used which registers and/or references and positionally tracks the tooth and/or jaw area, and wherein a magnetic field based navigation and/or tracking system is used which is coupled to it regarding the navigation and/or tracking, and which registers and/or references and positionally tracks the instruments or apparatus for treatment, using an external magnetic field and induction-sensitive elements (31, 32).

10. The method as set forth in any one of claims 1 to 9, wherein said three-dimensional volume data set is obtained by one or more of the following methods: computer tomography, nuclear spin tomography, PET, SPECT, ultrasonic imaging.

11. The method as set forth in any one of claims 1 to 10, wherein the treatment is further assisted by intra-operative imaging methods.

12. The method as set forth in any one of claims 1 to 11, wherein a screen output is provided, on the basis of one or more of the following representations:
- a two and three-dimensional representation of the registered and/or referenced and positionally determined tooth and/or jaw area as well as of the instruments including desired and actual positional courses;
- OPG views;
- digitally reconstructed radiographs (DRRs), in particular in the form of OPG views.

13. The method as set forth in any one of claims 1 to 12, wherein the treatment is carried out by instruments which are fully or partly guided by a robot which is registered and/or referenced and positionally tracked in the navigation and/or tracking system (10), and controlled by the system.

14. A device for assisting in a dental treatment, comprising a navigation and/or tracking system (10) which supplies information for assisting and/or guiding the treatment during the treatment by means of an output unit (16), also instruments and apparatus for treatment (40) which are registered and/or referenced relative to the computer-assisted navigation and/or tracking system (10) and positionally tracked, **characterised by** a device producing a three-dimensional volume data set for the tooth and/or jaw area of a patient by means of an imaging method, for example tomographic imaging, and registering and/or referencing the tooth and/or jaw area by means of a computer-assisted navigation and/or tracking system (10) which uses the volume data set, **characterised in that** it comprises an output device which outputs a visual and/or acoustic warning signal when the desired positional course and actual positional course deviate, whereupon a new desired positional course is calculated and outputted, in particular when the desired positional course and actual positional course deviate.

15. The device as set forth in claim 14, **characterised in that** it comprises a device with which the tooth and jaw area is also tracked by said navigation and/or tracking system.

16. The device as set forth in claim 14 or 15, **characterised in that** the output unit is a screen display (16), on which the tooth and/or jaw area, as well as the desired positional course of an instrument (40) planned to begin with or inter-operatively, is displayed during the treatment, and the actual positional course of the instrument (40) is positionally tracked.

17. The device as set forth in claim 16, **characterised in that** it comprises an optical navigation and tracking system which registers and/or references and positionally tracks the tooth and/or jaw area and/or the instruments or apparatus for treatment, using markers or arrangements of markers (31, 32) and a camera system, in particular an infrared navigation and/or tracking system.

18. The device as set forth in any one of claims 14 to 17, **characterised in that** it comprises a magnetic field based navigation and tracking system, which registers and/or references and positionally tracks the tooth and/or jaw area and/or the instruments or apparatus for treatment, using an external magnetic field and induction-sensitive elements (31, 32).

19. The device as set forth in any one of claims 14 to 18, **characterised in that** it comprises an optical navigation and tracking system which registers and/or references and positionally tracks the tooth and/or jaw area, and **in that** it comprises a magnetic field based navigation and tracking system coupled to it regarding navigation and/or tracking, which registers and/or references and positionally tracks the instruments or apparatus for treatment, using an external magnetic field and induction-sensitive elements (31, 32).

20. The device as set forth in any one of claims 14 to 19, **characterised in that** it further comprises devices which assist in the treatment with intra-operative imaging methods.

21. The device as set forth in any one of claims 14 to 20, **characterised in that** it comprises a robot which fully or partly guides the instruments during the treatment, is registered and/or referenced in the navigation and/or tracking system (10) and positionally tracked, and is controlled by the system.

## Revendications

1. Procédé d'assistance de traitement dentaire pour lequel
- un jeu de données spatiales tridimensionnelles est établi pour une zone dentaire ou maxillaire d'un patient à l'aide d'un procédé d'imagerie, par exemple d'une acquisition par tomographie,
- la zone dentaire ou maxillaire est enregistrée et référencée à l'aide d'un système de navigation et de suivi assisté par ordinateur (10) qui utilise le jeu de données spatiales,
- des instruments et appareils de traitement (40) sont enregistrés et référencés par rapport au système de navigation et de suivi assisté par ordinateur (10) et peuvent être suivis en position, et pour lequel
- le système de navigation et de suivi (10) fournit à l'aide d'une unité de sortie (16) des informations assistant ou guidant le traitement avant, pendant ou après le traitement, **caractérisé en ce que**
- en cas de déviations entre l'évolution de la position de consigne et l'évolution de la position réelle, une nouvelle évolution de la position de consigne est calculée et affichée.

2. Procédé suivant la revendication 1, pour lequel la zone dentaire et maxillaire est également suivie en position par le système de navigation et de suivi.

3. Procédé suivant la revendication 1 ou 2, pour lequel la zone dentaire et maxillaire ainsi que l'évolution de la position de consigne d'un instrument (40) prévue au préalable ou en cours d'opération sont affichées lors du traitement sur un écran d'affichage (16) et l'évolution de la position réelle de l'instrument (40) est suivie en position.

4. Procédé suivant la revendication 3, pour lequel un avertissement visuel et/ou acoustique est donné en cas de déviations entre l'évolution de la position de consigne et l'évolution de la position réelle.

5. Procédé suivant l'une des revendications 1 à 4, pour lequel on utilise un système de navigation et de suivi qui effectue l'enregistrement et le référencement d'un jeu de données comprenant des données tridimensionnelles pour la zone dentaire et maxillaire à l'aide d'un des procédés suivants:
- référencement et enregistrement du jeu de données à l'aide de points de repère anatomiques,
- référencement et enregistrement du jeu de données à l'aide de points de repère artificiels, en particulier de marqueurs à rayonnement actif ou réfléchissants,
- référencement et enregistrement du jeu de données à l'aide d'une technique de concordance de surface reposant sur une détection anatomique,
- référencement et enregistrement du jeu de données à l'aide d'une technique de concordance de surface reposant sur une détection sans contact, en particulier sur la génération de marquages de rayonnement laser sur la surface du patient.

6. Procédé suivant l'une des revendications 1 à 5, pour lequel le suivi positionnel de la zone dentaire et maxillaire se fait via une méthode de suivi dynamique, en particulier par un suivi dynamique simultané du maxillaire supérieur et/ou du maxillaire inférieur ou du maxillaire supérieur et du maxillaire inférieur respectivement pris individuellement, de préférence via des adaptateurs de référencement avec dispositifs marqueurs qui y sont fixés.

7. Procédé suivant l'une des revendications 1 à 6, pour lequel on utilise un système optique de navigation et de suivi qui effectue l'enregistrement et le référencement ainsi que le suivi de position de la zone dentaire et maxillaire et/ou des instruments et appareils de traitement avec utilisation de marqueurs et dispositifs marqueurs (31, 32) et d'un système de caméra, un système de navigation et de suivi infrarouge étant en particulier utilisé.

8. Procédé suivant l'une des revendications 1 à 7, pour lequel on utilise un système de navigation et de suivi basé sur un champ magnétique, qui effectue l'enregistrement et le référencement ainsi que le suivi de position de la zone dentaire et maxillaire et/ou des instruments et appareils de traitement avec utilisation d'un champ magnétique externe et d'éléments sensibles à l'induction (31, 32).

9. Procédé suivant l'une des revendications 1 à 8, pour lequel on utilise un système optique de navigation et de suivi, lequel effectue l'enregistrement et le référencement et le suivi de position de la zone dentaire et maxillaire et pour lequel on utilise un système de navigation et de suivi basé sur un champ magnétique qui y est couplé sur le plan de la navigation et du suivi, qui effectue l'enregistrement et le référencement ainsi que le suivi de position des instruments et appareils de traitement avec utilisation d'un champ magnétique extérieur et d'éléments sensibles à l'induction (31, 32).

10. Procédé suivant l'une des revendications 1 à 9, pour lequel le jeu de données spatiales tridimensionnelles est obtenu par l'un ou plusieurs des procédés suivants: tomographie assistée par ordinateur, tomographie par résonance magnétique nucléaire, PET, SPECT, acquisition par ultrasons.

11. Procédé suivant l'une des revendications 1 à 10, pour lequel le traitement est en outre assisté par des procédés d'imagerie en cours d'opération.

12. Procédé suivant l'une des revendications 1 à 11, pour lequel un affichage à l'écran est effectué sur base d'une ou plusieurs des représentations suivantes:
- représentation bidimensionnelle et tridimensionnelle de la zone dentaire et maxillaire enregistrée et référencée et déterminée en position ainsi que des instruments avec évolution de la position de consigne et de la position réelle,
- vues panoramiques (OPG),
- images radiographiques reconstituées par voie numérique (DRR = Digitally Reconstructed Radiographs), en particulier sous la forme de vues OPG.

13. Procédé suivant l'une des revendications 1 à 12, pour lequel le traitement est effectué à l'aide d'instruments qui sont guidés entièrement ou partiellement par un robot qui est enregistré et référencé dans le système de navigation et de suivi (10) et dont la position est suivie et qui est commandé par le système.

14. Dispositif d'assistance d'un traitement dentaire avec un système de navigation et de suivi (10) qui, lors du traitement, fournit à l'aide d'une unité de sortie (16) des informations assistant ou guidant le traitement et avec des instruments et appareils de traitement (40) qui sont enregistrés et référencés par rapport au système de navigation et de suivi assisté par ordinateur (10) et sont suivis en position, avec un dispositif qui, à l'aide d'un procédé d'imagerie, par exemple un balayage par tomographie, établit un jeu de données spatiales tridimensionnelles pour la zone dentaire et maxillaire d'un patient et enregistre et référence la zone dentaire et maxillaire à l'aide du système de navigation et de suivi (10), qui utilise le jeu de données spatiales, **caractérisé en ce qu'**il présente un dispositif d'affichage qui donne un avertissement visuel et/ou acoustique en cas de déviations entre l'évolution de la position de consigne et l'évolution de la position réelle, suite à quoi, en particulier en cas de déviations entre l'évolution de la position de consigne et l'évolution de la position réelle une nouvelle évolution de la position de consigne est calculée et affichée.

15. Dispositif suivant la revendication 14, **caractérisé en ce qu'**il présente un dispositif avec lequel la zone dentaire et maxillaire est également suivie en position par le système de navigation et de suivi.

16. Dispositif suivant la revendication 14 ou 15, **caractérisé en ce que** l'unité de sortie est un écran d'affichage (16) sur lequel la zone dentaire et maxillaire ainsi que l'évolution de la position de consigne d'un instrument (40) prévue au préalable ou en cours d'opération sont affichées lors du traitement et l'évolution de la position réelle de l'instrument (40) est suivie en position.

17. Dispositif suivant la revendication 16, **caractérisé en ce qu'**il présente un système optique de navigation et de suivi qui effectue l'enregistrement et le référencement ainsi que le suivi de position de la zone dentaire et maxillaire et/ou des instruments et appareils de traitement avec utilisation de marqueurs et dispositifs marqueurs (31, 32) et d'un système de caméra, en particulier un système de navigation et de suivi infrarouge.

18. Dispositif suivant l'une des revendications 14 à 17, **caractérisé en ce qu'**il présente un système de navigation et de suivi basé sur un champ magnétique, qui effectue l'enregistrement et le référencement ainsi que le suivi de position de la zone dentaire et maxillaire et/ou des instruments et appareils de traitement avec utilisation d'un champ magnétique externe et d'éléments sensibles à l'induction (31, 32).

19. Dispositif suivant l'une des revendications 14 à 18, **caractérisé en ce qu'**il présente un système optique de navigation et de suivi, lequel effectue l'enregistrement et le référencement et le suivi de position de la zone dentaire et maxillaire et **en ce qu'**il présente un système de navigation et de suivi basé. sur un champ magnétique qui y est couplé sur le plan de la navigation et du suivi, qui effectue l'enregistrement et le référencement ainsi que le suivi de position des instruments et appareils de traitement avec utilisation d'un champ magnétique extérieur et d'éléments sensibles à l'induction (31, 32).

20. Dispositif suivant l'une des revendications 14 à 19, **caractérisé en ce qu'**il présente en outre des dispositifs avec lesquels le traitement est assisté par des procédés d'imagerie en cours d'opération.

21. Dispositif suivant l'une des revendications 14 à 20, **caractérisé en ce qu'**il présente un robot qui guide les instruments entièrement ou partiellement lors du traitement, qui est enregistré et référencé dans le système de navigation et de suivi (10) et dont la position est suivie et qui est commandé par le système.
